(19) **Europäisches Patentamt / European Patent Office / Office européen des brevets**

(11) **EP 1 835 851 B1**

(12) **EUROPEAN PATENT SPECIFICATION**

(45) Date of publication and mention of the grant of the patent:
**25.06.2014 Bulletin 2014/26**

(51) Int Cl.:
***A61B 5/021*** (2006.01)

(21) Application number: **06700154.5**

(86) International application number:
**PCT/GB2006/000008**

(22) Date of filing: **04.01.2006**

(87) International publication number:
**WO 2006/072776 (13.07.2006 Gazette 2006/28)**

(54) **A METHOD OF OBTAINING AN ESTIMATION OF A PERSON'S AORTIC BLOOD PRESSURE**

VERFAHREN ZUR GEWINNUNG EINER EINSCHÄTZUNG DES ARTERIELLEN BLUTDRUCKS EINER PERSON

PROCEDE PERMETTANT D'OBTENIR UNE ESTIMATION DE LA PRESSION SANGUINE AORTIQUE D' UNE PERSONNE

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.01.2005 GB 0500430**

(43) Date of publication of application:
**26.09.2007 Bulletin 2007/39**

(73) Proprietor: **CAREFUSION U.K. 235 LIMITED Basingstoke, Hampshire RG22 4BS (GB)**

(72) Inventors:
 • **CHOWIENCZYK, Philip, Jan London SE3 0QH (GB)**
 • **MILLASSEAU, Sandrine Morden, Surrey SM4 5LT (GB)**

(74) Representative: **Richards, John et al Ladas & Parry LLP Dachauerstrasse 37 80335 München (DE)**

(56) References cited:
**US-A- 5 265 011**

 • **MILLASSEAU SANDRINE C ET AL: "Pressure wave reflection assessed from the peripheral pulse: is a transfer function necessary?" HYPERTENSION. MAY 2003, vol. 41, no. 5, May 2003 (2003-05), pages 1016-1020, XP002370569 ISSN: 1524-4563**
 • **SIEBENHOFER A ET AL: "The reproducibility of central aortic blood pressure measurements in healthy subjects using applanation tonometry and sphygmocardiography." JOURNAL OF HUMAN HYPERTENSION. SEP 1999, vol. 13, no. 9, September 1999 (1999-09), pages 625-629, XP008060928 ISSN: 0950-9240**

**Description**

This invention relates to a method of obtaining an estimation of a person's aortic blood pressure.

Sustained high blood pressure is known as hypertension and it is a major cause of cardio vascular disease and death. A person's blood pressure is usually measured non-invasively. Typically, a sphygmomanometer is employed. The sphygmomanometer is externally placed around a person's arm, inflated and then the person's brachial blood pressure is read.

It is recognised that the measurement of aortic systolic blood pressure may be superior to that of brachial systolic blood pressure because blood pressure in the aorta is more closely related to the load imposed on the heart. Measurement of the aortic blood pressure rather than the brachial blood pressure may identify subjects that are at increased risk of cardiovascular disease. The measurement of the aortic systolic blood pressure may also identify person's with pseudo systolic hypertension in whom brachial systolic (maximum) blood pressure is high but aortic systolic blood pressure is normal. Assessment using brachial blood pressure alone may lead to inappropriate treatment for a person. In spite of all this, the aortic blood pressure is often not obtained because the measurement of the aortic blood pressure can usually only be made in an invasive manner using a pressure catheter in the person's aorta. Such measurements can only generally be made In a hospital and they are therefore not normally conduced due to the expense, discomfort and possible side effects imposed by such a procedure.

US-A-5285011 and EP-A-1380254 described methods of obtaining an estimation of a person's aortic systolic blood pressure non-invasively by applying a transfer function to the pressure waveform measured from the radial artery of the person. Pressure wave reflection assessed from the peripheral pulse: is a transfer function necessary?', Millasseau Sandrine C.et al., U, May 2003, ISSN: 1524-4563, vol. 41, no. 5, pg. 1016-1020 relates to transfer functions.

UK-A-0317847.2 describes a method of estimating a person's aortic systolic blood pressure by applying a transfer function to a blood pressure pulse measured at an extremity of the person using a photoplethysmograph.

The above mentioned three methods of estimating the person's aortic systolic blood pressure use a transfer function derived from data on a subset of persons. These methods assume that the transfer function is applicable to all persons. The validity of this assumption and the accuracy of the transfer function may be questioned.

It Is an aim of the present invention to provide a method of obtaining an estimation of a person's aortic systolic blood pressure, which method does not require the use of a transfer function, and which method also does not require the use of a pressure catheter in the person's aorta.

Accordingly, the present invention provides a method of obtaining an estimation of a person's aortic systolic blood pressure, which method comprises non-invasively measuring the person's brachial systolic blood pressure, non-invasively measuring the person's brachial diastolic blood pressure, obtaining the person's radial augmentation index by non-invasively measuring the person's radial pulse waveform, and obtaining the estimation of the person's aortic systolic blood pressure from the following equation:

$$aSBP = \alpha + \beta . rA1x + \gamma . bSBP + \delta . bDBP \qquad (1)$$

where the coefficients $\alpha$, $\beta$ $\gamma$ and $\delta$ are constants with approximate values of

$\alpha$ = -24.2
$\beta$ = 0.28
$\gamma$ = 0.83
$\delta$ = 0.17

The radial augmentation index is the ratio of the second systolic peak to the person's radial pulse pressure (systolic pressure - diastolic pressure). The use of the equation gives an estimation of the person's aortic systolic blood pressure that is extremely accurate, for example to within 3mm Hg.

In the above equation, the final value given for the coefficients $\alpha$, $\beta$ $\gamma$ and $\delta$ may vary slightly in order to provide a more accurate value of the person's aortic systolic blood pressure.

The method may be one in which the person's brachial systolic blood pressure is non-invasively measured using a sphygmomanometer. Other devices for non-invasively measuring the person's brachial systolic blood pressure may be employed.

The method may be one in which the person's brachial diastolic blood pressure is non-invasively measured using a sphygmomanometer. Other devices for non-invasively measuring the person's brachial diastolic blood pressure may be employed.

The method may be one in which the person's radial pulse waveform is measured using a tonometer. Other devices for non-invasively measuring the person's radial pulse waveform may be used.

An embodiment of the invention will now be described solely by way example and with reference to the accompanying drawings in which:

Figure 1 is a graph of a radial pressure pulse and shows how to obtain the radial augmentation index as a ratio of the second systolic peak to the pulse pressure; and

Figure 2 shows apparatus for measuring a person's brachial systolic blood pressure and a person's brachial diastolic blood pressure.

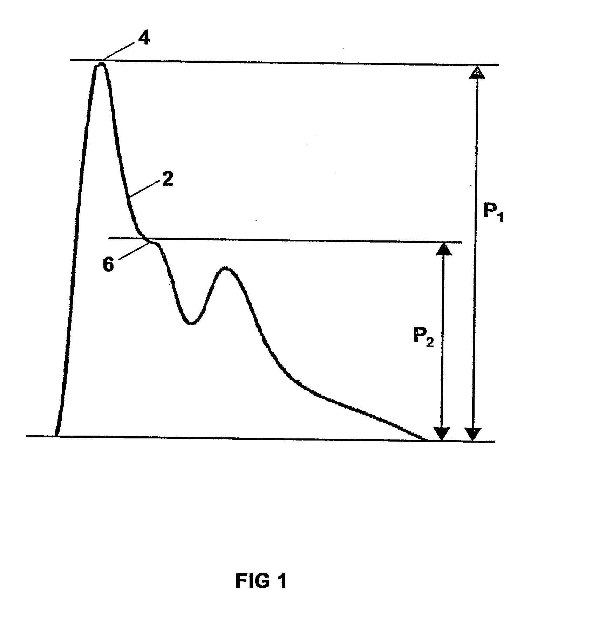

Referring to Figure 1, there is shown a radial pressure pulse having a first systolic peak 4, and a second systolic peak 6. The second systolic peak 6 appears somewhat as a shoulder. The first systolic peak 4 gives a pressure $P_1$, and the second systolic peak 6 gives a pressure $P_2$. The radial augmentation index is defined as the ratio of the second systolic peak 6 to the pressure pulse and may thus be defined by the following equation:

$$rAIx = P_2/P_1 \qquad\qquad (2)$$

where rAIx = radial augmentation index $P_1$ = the first systolic peak, and $P_2$ = the second systolic peak.

The aortic systolic blood pressure is estimated to a high degree of accuracy using the above mentioned equation No. (1).

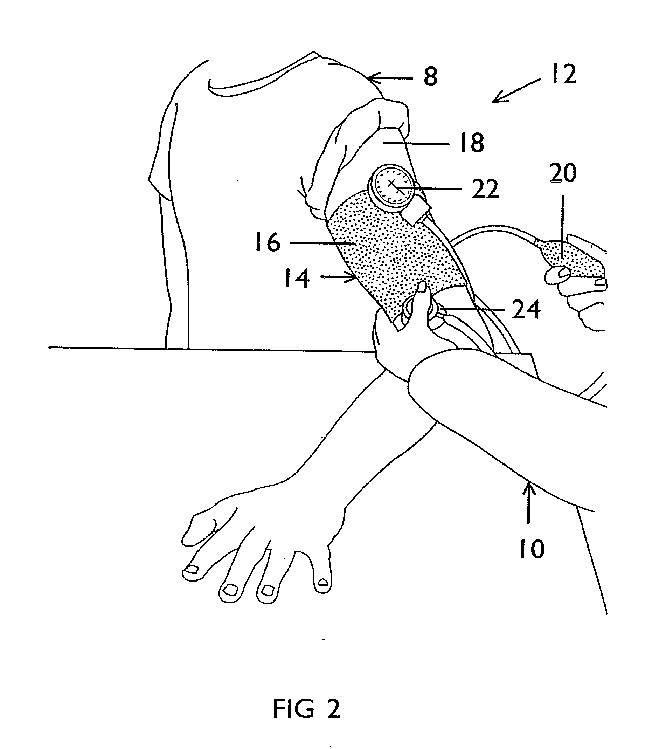

Referring now to Figure 2, there is shown a person having their brachial systolic blood pressure or their brachial diastolic blood pressure measured by a person 10. The person 10 is using apparatus 12 including a sphygmomanometer 14. The sphygmomanometer 14 comprises a cuff 16 which is placed around an arm 18 of the person 8. The cuff 16 is inflated using a hand pump 20. The pressure in the cuff 16 is shown on a dial 22. The apparatus 12 also comprises a stethoscope 24 which is placed over the brachial artery. By listening to the stethoscope during deflation of the cuff 16, is it possible to identify the pressure corresponding to systolic and diastolic blood pressure.

It is to be appreciated that the embodiment of the invention described above with reference to the accompanying drawings has been given by way of example only and that modifications may be affected. Thus, for example, the coefficients $\alpha$, $\beta$, $\delta$, and $\gamma$ may be constants of slightly different values.

**Claims**

1. A method of obtaining an estimation of a person's aortic systolic blood pressure, which method comprises non-invasively measuring the person's brachial systolic blood pressure, non-invasively measuring the person's brachial diastolic blood pressure, obtaining the person's radial augmentation index by non-invasively measuring the person's radial pulse waveform, and obtaining the estimation of the person's aortic systolic blood pressure from the following equation:

$$aSBP = \alpha + \beta. rA1x + \gamma. bSBP + \delta. bDBP$$

where the coefficients $\alpha$, $\beta$, $\gamma$ and $\delta$ are constants with approximate values of

$\alpha = -24.2$
$\beta = 0.28$
$\gamma = 0.83$
$\delta = 0.17$

2. A method according to claim 1 in which the person's brachial systolic blood pressure is non-invasively measured using a sphygmomanometer.

3. A method according to claim 1 or claim 2 in which the person's brachial diastolic blood pressure is non-invasively measured using a sphygmomanometer.

4. A method according to claim 1 in which the person's radial pulse waveform is obtained using a tonometer.


**Patentansprüche**

1. Verfahren zum Erhalten einer Schätzung des systolischen, arteriellen Blutdrucks einer Person, wobei das Verfahren das nicht invasive Messen des systolischen Blutdrucks der Person am Oberarm, das nicht invasive Messen des diastolischen Blutdrucks der Person am Oberarm, das Erhalten des radialen Augmentationsindex der Person durch nicht invasives Messen der Radialpulswellenform und das Erhalten der Schätzung des systolischen, arteriellen Blutdrucks der Person aus folgender Gleichung umfasst:

$$aSBP = \alpha + \beta . rA1x + \gamma . bSBP + \delta . bDBP,$$

wobei die Koeffizienten $\alpha$, $\beta$, $\gamma$ und $\delta$ Konstanten mit den folgenden ungefähren Werten sind:

$\alpha = -24,2$
$\beta = 0,28$
$\gamma = 0,83$
$\delta = 0,17.$

2. Verfahren nach Anspruch 1, wobei der systolische Blutdruck der Person am Oberarm nicht invasiv unter Verwendung eines Sphygmomanometers gemessen wird.

3. Verfahren nach Anspruch 1 oder Anspruch 2, wobei der diastolische Blutdruck der Person am Oberarm nicht invasiv unter Verwendung eines Sphygmomanometers gemessen wird.

4. Verfahren nach Anspruch 1, wobei die Radialpulswellenform der Person unter Verwendung eines Tonometers erhalten wird.


**Revendications**

1. Procédé permettant d'obtenir une estimation de la pression sanguine systolique aortique d'une personne, lequel procédé comprend les étapes consistant à mesurer de manière non invasive la pression sanguine systolique brachiale de la personne, mesurer de manière non invasive de la pression sanguine diastolique brachiale de la personne, obtenir l'indice d'augmentation radiale de la personne en mesurant de manière non invasive la forme d'onde d'impulsion radiale de la personne, et obtenir l'estimation de la pression sanguine systolique aortique de la personne à partir de l'équation suivante :

$$aSBP = \alpha + \beta . rA1x + \gamma . bSBP + \delta . bDBP$$

dans laquelle les coefficients $\alpha$, $\beta$, $\gamma$ et $\delta$ sont des constantes ayant les valeurs approximatives suivantes

$\alpha = -24,2$
$\beta = 0,28$
$\gamma = 0,83$
$\delta = 0,17$

2. Procédé selon la revendication 1, dans lequel la pression sanguine systolique brachiale de la personne est mesurée de manière non invasive à l'aide d'un sphygmomanomètre.

3. Procédé selon la revendication 1 ou 2, dans lequel la pression sanguine diastolique brachiale de la personne est mesurée de manière non invasive à l'aide d'un sphygmomanomètre.

**4.** Procédé selon la revendication 1, dans lequel la forme d'onde d'impulsion radiale de la personne est obtenue à l'aide d'un tonomètre.

**FIG 1**

FIG 2

**EP 1 835 851 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5285011 A
- EP 1380254 A
- GB 0317847 A